(19) **Europäisches Patentamt**
**European Patent Office**
**Office européen des brevets**

(11) **EP 1 364 637 B1**

(12) **EUROPÄISCHE PATENTSCHRIFT**

(45) Veröffentlichungstag und Bekanntmachung des Hinweises auf die Patenterteilung:
**12.01.2011 Patentblatt 2011/02**

(21) Anmeldenummer: **02707364.2**

(22) Anmeldetag: **23.01.2002**

(51) Int Cl.:
**A61H 7/00** *(2006.01)*  **A61H 15/02** *(2006.01)*

(86) Internationale Anmeldenummer:
**PCT/RU2002/000057**

(87) Internationale Veröffentlichungsnummer:
**WO 2002/058783 (01.08.2002 Gazette 2002/31)**

(54) **METHODE FÜR DIE BIOMECHANISCHE WELLENTHERAPIE**

METHOD FOR WAVE BIOMECHANICAL THERAPY

PROCEDE DE THERAPIE BIOMECANIQUE PAR ONDES

(84) Benannte Vertragsstaaten:
**AT BE CH CY DE DK ES FI FR GB GR IE IT LI LU MC NL PT SE TR**

(30) Priorität: **24.01.2001 RU 2001102239**

(43) Veröffentlichungstag der Anmeldung:
**26.11.2003 Patentblatt 2003/48**

(73) Patentinhaber:
• **Saveliev, Boris Sergeevich**
  **Magnitogorsk, 445004 (RU)**
• **Saveliev, Vladimir Sergeevich**
  **Samara, 443013 (RU)**
• **Zinkovich, Wladlen Ivanovich**
  **Toliyatti, 445031 (RU)**

(72) Erfinder:
• **SAVELIEV, Boris Sergeevich**
  **Magnitogorsk, 445004 (RU)**

• **SAVELIEV, Vladimir Sergeevich**
  **Samara, 443013 (RU)**
• **SKOVORODNIKOV, Wladimir Wasilievich**
  **Moskovskaya obl., 141060 (RU)**
• **ZINKOVICH, Wladlen Ivanovich**
  **Toliyatti, 445031 (RU)**
• **GOLEV, Nikolay Nikolaevich**
  **Toliyatti, 445000 (RU)**

(74) Vertreter: **Jeck, Anton**
**Jeck - Fleck - Herrmann**
**Patentanwälte**
**Klingengasse 2/1**
**71665 Vaihingen/Enz (DE)**

(56) Entgegenhaltungen:
**EP-A2- 0 784 997     WO-A1-00/67693**
**DE-C1- 3 905 517     RU-C1- 2 014 056**
**RU-C1- 2 045 972     RU-C2- 2 161 517**
**SU-A3- 1 795 889     US-A- 5 746 702**

EP 1 364 637 B1

**Beschreibung**

Die Erfindung definiert in Ausruch 1 gehört zum Medizin-, Sport- und Kosmonautikbereich.

Der Kern der Offenbarung besteht darin, dass in dem vorgeschlagenen Verfahren zur Biomechanotherapie die Massage- und therapeutische Einwirkung auf den Menschenorganismus sowohl durch Wärme- und Lichtwellen, als auch durch mechanische Wellen erfolgt, die eine aufeinanderfolgende und parallele Kombination der modulierten und einzelnen Längs- und Querwellen mit einer Länge von 0,005 bis 0,1 m darstellen und die sich auf dem Menschenkörper mit einer Geschwindigkeit von 0,01 bis 12 m/s fortpflanzen; dabei lassen sich einsame Wellen auf dem Körper durch die Impulskraft der einzelnen Thermovibratode bilden, die untereinander durch eine steuerbare Bindung verbunden sind und auf den Menchenkörper bei 0° bis 90° C, mit einem spezifischen Druck von $0,5 \times 10^5$ bis $4 \times 10^5$ Pa und einer Druckkraft von 0,1 bis 100H einwirken. Mit dem vorgeschlagenen Verfahren der Biomechanotherapie lässt sich die Effektivität der Kur- und Sportmassage erhöhen und lassen sich auch Ergebnisse der komplexen Heilung von unterschiedlichen Erkrankungen verbessern. Die Wellenbiomechanotherapie erhöht den peripherischen Blutumlauf um 1,5-2fache, indem die Arbeit des Herz-Vaskulösesystems erleichtert und die reologischen Bluteigenschaften und Blutversorgung aller Organe und ihre funktionelle Zustände verbessert werden. Die Wellenbiomechanotherapie stimuliert aktiv den Stoffwechsel, beschleunigt Wiederherstellungsprozesse in Organen und Geweben und verstärkt die Wegführung der Zerfallprodukte aus dem Organismus auf natürlichem Wege.

Die Erfindung gehört zum Gebiet der physischen Kultur und Sport, Medizin und Kosmonautik und kann sowohl zur Gesundungsmassage, als auch zur Kurmassage ausgenutzt werden sowie für die Heilung verschiedener Erkrankungen.

Bekannt ist ein Verfahren zur pneumatischen Massage unter Anwendung von Hüllen, in der die harmonische Wanderwelle ausgenutzt wird (Patent US Nr. 4231355). Dieses Verfahren stellt die Einwirkung nur durch die Transversallwelle sicher, die sich geradlinig fortgepflanzt, was der Mehrheit der Massagehandgriffe nicht entspricht, die nach dem ungeradlinigen Wege ausgeführt werden, die zum Beispiel durch Blut- und Lymphumlauf des massierten Körperteils bedingt sind.

Bekannt ist die Erfindung der Kurmassage, die Wanderwellen benutzt (Urheberschein UdSSR Nr. 1795889). Diese Einrichtung bildet nur mechanische Querwellen, die sich geradlinig fortpflanzen, für eine begrenzte Anzahl von anatomtopographischen Körperteilen. Der Mangel an Wärme-, Licht- und Unterdrückwellen und eine Impulsbewegung der Module der pseudokochenden Schicht der Körperoberfläche entlang lässt keine effektive Einwirkung auf den Lymphumlauf zu. In dieser bekannten Einrichtung fehlen Möglichkeiten für den Aufbau einer modulierten Welle, die eine wichtige Kennlinie der einsamen Welle ist, wegen der Anwendung in der Einrichtung des Antriebsmoduls für die Rotation von Scheiben. Einrücken und Antriebarbeit mit der Absicht des Schaffens einer choatischen Bewegung der Kügelchen und das Motorausrücken fordern viel Zeit, d.h. der Prozess der Bildung der kochenden Schicht ist inert. Insbesondere wird darauf hingewiesen, dass sich unter dem Regime der "Wanderwelle" Module auf 5-10 Sekunden beschränken lassen, was der Geschwindigkeit der Wellenfortpflanzung entspricht, die mit der Geschwindigkeit der Lymphbewegung kommen surabel ist.

Bekannt ist ein Verfahren zur Massage (Internationales Patent Nr. WO 00/67693). Dieses Verfahren realisiert nur die mechanische Wanderwelle am liegenden Körper entlang. Mit der Wellenbildung werden vier Vibratoden ausgenutzt, die nicht auf einem biomechanischen Glied liegen, d.h. die Wellenlänge ist viel größer als 0,1 m. Wärme- und Lichtwellen fehlen. Parameter der Modulation der mechanischen Impulse sind dieselben für alle Vibrationsquellen. Aus Mangel an einer steuerbaren Verbindung zwischen den Vibratoden und an großer Wellenlänge lässt sich eine Ringmassage nicht durchführen, bei der ein Umgreifen eines Körperteils möglich ist, das sowohl den Blutumlauf stimuliert, als auch diesen kurzzeitig völlig unterbindet. Die Trägerfrequenz bei diesem Verfahren entspricht dem Frequenzbereich (5-20Hz) der biomechanischen Resonanz nicht, und infolgedessen kann der Blutumlauf in den Muskeln direkt nicht beeinflusst werden.

Bekannt ist eine befeuchtende, erwärmende, vibrierende, elektrisierende und beleuchtende Kugelmassagerolle (DE 3905517 C1). Diese Rolle sichert die Kombination aller wirkenden Arbeitsfaktoren nicht, d.h. eine Folge von Wärme, Vibration, Beleuchtung mit verschiedenen Zeitinterwallen. Bei bekannten Rollen wird ein Vibrationsmotor benutzt, der wie alle elektrischen Antriebe eine hohe Trägheit und ein Verhältnis von 2 hat. Infolgedessen fehlt die Möglichkeit, jeden mechanischen Impuls zu programmieren und die Modulation der mechanischen Impulse unter Aufnahme vom Organismus zu verwirklichen. Die Konstruktion der Einrichtung lässt nicht zu, die Ringmassage nicht durchzuführen und Phasen der mechanischen, thermischen und Lichtimpulse zu synchronisieren.

In die Nähe der vorliegenden Erfindung kommt insbesondere ein Verfahren zur Wellentherapie unter Benutzung von Zylinderhüllen, die parallel zueinander liegen und an einen Menschenkörper gedrückt sind (Urheberschein UdSSR Nr. 604211). Die Wellenbewegung der elastischen Oberfläche durch abwechselndes Anfüllen und Wegnehmen der Hüllen lässt für die Massage eine flache harmonische Wanderwelle entstehen, die mit der Geschwindigkeit der Impulswelle in den Gliedmaßen synchronisiert ist.

Der Mangel an Druckkraft schließt bei diesem Massageverfahren die Verwendung von Streichhandgriffen aus, für die Regulierung bei dem Therapieprozess des Haut- und Muskeltonus notwendig ist. Außer der wellenförmigen Bewegung, bei der die Wirkungsformen für alle Körperteile gleich sind, erzeugt die harmonische Wanderwelle eine schnelle Adaption des Organismus, bei der die Aktivität des Nerven- und Muskelapparats abnimmt. Das alles hat eine Wirkung auf die Wiederherstellung des Organismus während der Einwirkung der mechanischen Impulse. Das zeigt sich in einer kurzen Prozedurdauer (15-30 Minuten). Der einzig regulierende Parameter, mit dessen Veränderung die Adaptation vermieden werden könnte, ist nur die Wellengeschwindigkeit. So fehlt die Möglichkeit, die Kurwirkung in breiten Grenzen zu regeln. Der weitere Nachteil dieses Verfahrens ist der Mangel an einer Möglichkeit, durch die Welle auf Vibrationsrezeptoren und wellenbiomechanische Prozesse in Miofibrillen zu wirken. Außerdem lässt dieses Verfahren nicht zu, die Wellen unter Unterdruck zu realisieren, das heißt, die Welle mit einem Druck weniger als $1{,}0 \times 10^5$ Pa zu verwenden.

Dokumente RU2045972 und RU2161517 offenbaren Vorrichtungen die Behandlungen mittels licht, bzw. Laser licht, und Druck durchführen.

Die Aufgabe der Erfindung ist die Erhöhung der Wirkung der Biomechanotherapie durch Schaffung einer Biomechanotherapiemethode, die verschiedene Massagehandgriffe durch Verbindung und Kombination der Einwirkung auf den Organismus verschiedener modulierter Wellen (Wärme- und Lichtwellen und mechanische Wellen) realisiert. Die Wellenverbindung nimmt die gleichzeitige Wirkung von mehreren Wellentypen an, und bei der kombinierenden Wirkung werden Wärme- und Lichtwellen und mechanische Wellen in der Therapie konsequent mit unterschiedlichen Zeitinterwallen verwendet.

Diese Aufgabe wird durch Auspruch 1 gelöst. Bei dem Verfahren der Biomechanotherapie wird die therapeutische Wirkung entweder durch Verbindung und Kombination der Wärme- und Lichtwellen und mechanische Wellen verwirklicht, die eine aufeinanderfolgende und parallele Kombination von Längs- und Querwellen und modulierten Einzelwellen mit einer Länge von 0,005 bis 0,1 m darstellen, die sich über den Körper mit einer Geschwindigkeit von 0,01 bis 12 m/cek fortpflanzen. Die einzelenen Längswellen bilden dabei infolge der Impulsbewegung einzelner Thermovibratode längs der Körperoberfläche auf dem Körper, senkrecht zur Körperoberfläche Längs- und Querwellen, wobei die Vibratode untereinander mit einer steuerbaren Verbindung verbunden sind und auf den Menschenkörper bei 0° bis 90° C mit einem spezifischen Druck von $0{,}5 \times 10^5$ bis $4 \times 10^5$ Pa, mit einer Druckkraft von 0,1 bis 100 H und einer Dauer von 1 Minute bis 10 Stunden wirken.

Modulierende Schwingungen der einzelnen Wellen sind Impulse mit der Frequenz von 0,004 bis 1 H und einem Verhältnis, das der Anzahl der Vibratode entspricht, die an der Wellenbildung teilnehmen, und Trägerschwingungen sind eine Reihenfolge von Impulsen mit der Frequenz von 1 bis 40 H und einem Verhältnis von 1,1 bis 6, bei denen die Frequenzmodulation zum Beispiel durch ein Sinussignal verwirklicht wird, bei dem sich die Frequenzmodulation von 0,004 bis 1 H und die Frequenzabweichung von 0,001 bis 40 Hz verändert. Jeder Vibratode hat einen Ausstrahler, der mit Hilfe eines faseroptischen Lichtleiters mit einer eigenen Laserausstrahlungsquelle verbunden ist, deren Beleuchtungsintensität mit der Phase der Schwingungen der thermomechanischen Impulse synchronisiert ist, und alle Quellen insgesamt dienen zur Bildung der einzelnen Welle der Laserstrahlung auf die Körperoberfläche. In alle Vibratoden wird Luft mit einer Temperatur von 0° bis 90° C gegeben.

Der Kern der offenbarung besteht darin, bei der Therapie aufeinanderfolgende und parallele Wellenkombinationen zu verwenden. Bei der traditionellen Kurmassage existiert ein konsequentes Verfahren zur Informationsübergabe, die sich in der konsequenten Ausführung der Kurmassagehandgriffe ausdrückt. Die gleichzeitige Durchführung von mehreren Handgriffen der Massage, bei der manuellen Massage ist durch die physischen Möglichkeiten des Masseurs, d.h. auf nicht mehr als zwei, begrenzt, und lässt sich bei der bekannten Apparatmassage nicht vorsehen. Infolge der parallelen Wellenkombination ist es möglich, die Massage über die ganze Oberfläche des Menschenkörpers unter Verwendung einer gleichzeitigen Stimulierung einiger Körperteile des Arterienumlaufes - und auf den anderen - entweder eines Lymphumlaufs oder eines Venenablaufs, durchzuführen. Bei dem vorgeschlagenen Verfahren zur Biomechanotherapie kann man eine bedeutende Anzahl von parallel durchgeführten Massagehandgriffen (Wellentypen) und dabei auf demselben Körperteil durchführen. Die bei der vorliegenden Erfindung modulierte Einzelwelle ist eine Wellenbewegung, die in jedem Zeitpunkt im Endbereich des Raumes lokalisiert ist und wegen der Modulation ihre Struktur (Amplitude, Phase und Frequenz) bei der Fortpflanzung verändert. Die an den Körper angedrückten Thermovibratode erzeugen eine wegen des Impulsbewegung senkrecht zur Menschenkörperoberfläche stehende Querwelle und eine wegen der Impulsbewegung längs der Körperoberfläche wirkende Längswelle. Die Temperatureinwirkung während des Anfüllens einer Vibratode schafft Bedingungen für ein Durchdringen der Wärme in tiefliegendes Menschengewebe. Infolge der Erwärmung der massierten Körperteile entsteht eine Wärmewelle, die sich über die Körperoberfläche fortpflanzt. Impulsförmige, durch die Haut gehende Blutbestrahlung ruft eine Lichtwelle hervor, deren Durchdringungstiefe von dem Druck der Vibratode auf den Körper und der Bestrahlungsintensität abhängt, und infolge der aufeinanderfolgenden Bestrahlung der verschiedenen Körperteile wird eine Lichtwelle erzeugt, die sich über die Körperoberfläche fortpflanzt. Unter der Bedingung, dass die Parameter der mechanischen Impulse von unterschiedlichen Thermovibratoden anders sind und zu dem Zweck gewählt werden, eine Wellenbewegung nach dem einzelnen Wellentyp zu bilden, verhält sich die entstandene Wanderwelle wie ein Teilchen, und ihre Energie wird im Massagehandgriff je nach der Verschiebung realisiert. Die Wellenlänge wird in Abhängigkeit vom massierten Körperteil vom benutzten Massagehandgriff und von den geometrischen Ausmaßen der Vibratode bestimmt.

Es ist festgestellt worden, dass die Wellenbewegung wirksam realisiert werden kann, wenn auf dem biomechanischen Glied nicht weniger als vier unbewegliche Vibratoden oder nur eine, aber verstellbare Vibratode verwendet werden bzw. wird. Mit der Erhöhung der Anzahl der Vibratoden erhöht sich auch die Wirksamkeit der Massage. Die maximal mögliche Wellenlänge wird durch die Größe der pneumatischen Hülle bestimmt, die als Vibratode benutzt wird. Es ist festgestellt worden, dass bei der Körpermassage als größtes biomechanisches Menschenglied der maximale Durchmesser der Hülle nicht mehr als 0,1 m sein soll. Analoge Untersuchungen der Gesichtsmuskeln, die minimale Ausmaße haben, haben gezeigt, dass optimale Vibratodenausmaße 0,005 m sind. Daher wurde die Länge der Einzelwelle mit 0,005 bis 0,1 m gewählt.

Die Geschwindigkeit der Wellenfortpflanzung wird in folgender Weise bestimmt. Es ist bekannt, dass die durchschnittliche Geschwindigkeit der Lymphbewegung 0,01 m/s und die linearen Geschwindigkeiten des Blutumlaufs in den Arterien 0,4 m/s in Arteriolen 0,2 m/s, in Kapillaren 0,05 m/s, in Venulen 0,1 m/s und in Venen 0,2 m/s beträgt. Aber die Durchführungsgeschwindigkeit der Anregung von Druckrezeptoren nach den postganglionären Fasern des vegetativen Nervensystems beträgt 0,5 - 2 m/s. Die Geschwindigkeit der Anregungsdurchführung nach anderen Nervenfasern beträgt von 3 bis 120 m/s. Es ist logisch, eine minimale Geschwindigkeit der Einzelwelle zu wählen, die der Geschwindigkeit der Lymphbewegung entspricht. (0,01 m/s). Die maximale Geschwindigkeit wurde durch die Aufgabe der Stimulierung des Blutkreislaufes in verschiedenen Abteilungen des Gefäßbettes, durch die Geschwindigkeit der Anregungsdurchführung von den Druckrezeptoren und durch die Geschwindigkeit der biomechanischen Wellenprozesse in Miofibrillen bestimmt. Was die Wirkgröße auf den Blutumlauf und die Druckrezeptoren betrifft, reicht dafür die Wellengeschwindigkeit von 2 m/s aus. In Bezug auf die Stimulierung der Wellenprozesse in Miofibrillen reicht dieser Wert nicht aus. Deshalb wurde folgende Rechnung erstellt: die Geschwindigkeit der Einzelwelle ohne Frequenzmodulation beträgt:

$$V = d/T_H, \text{ aber } f = 1/(q \times T_H), \ T_H = 1/(q \times f),$$

das bedeutet V = qxdxf, wobei

d = lineares Ausmaß der Hülle in Richtung der Wellenfortpflanzung

$T_H$ = die Zeit des Anfüllens der Hülle

q = das Verhältnis der mechanischen Impulse

f = die Frequenz der Schwingungseinwirkung auf die Muskeln bei gleichzeitigem Anfüllen und Auslaufen der Hülle (bei q = 2)

Es ist experimentell festgestellt worden, dass die Vibrationsmassage der Muskeln mit Frequenzen bis 40 Hz und einem Verhältnis von 1,1 bis 6 die Wiederherstellungsvorgänge in Muskeln stimuliert. Die Verwirklichung der Vibration mit einer Frequenz von 40 Hz gelang unter Benutzung einer Hülle mit einem Durchmesser von d=0,0 m. Es folgt daraus, dass die maximale Wellengeschwindigkeit 12 m/s ist und durch Gleichung ausgedrückt werden kann:

$$Vmax = q_{max} \times d_{max} \times f_{max} = 6 \times 0{,}05 \times 40 = 12 \ m/s$$

Diese Geschwindigkeit der Bewegung des elastischen Mediums lässt die Wellenmassage mit der Geschwindigkeit der pulsierenden Welle der Gefäße des Muskeltyps synchronisieren.

Bei dem vorgeschlagenen Massageverfahren ist eine Vielzahl von Verschiebungsrichtungen der Einzelwellen (des Lymph- und Blutkreislaufs der Nervenund Reflexröhren und der Verbreitungswege der Muskelkraft entlang, d.h. den Muskelfasern entlang möglich. Daher lässt sich die nötige Richtung wählen und wegen der Wellenparameter der eine oder andere physiologische Mechanismus stimulieren. Die hauptsächliche Richtung der Wellenfortpflanzung, die biomechanischen Meridiane, ist durch Linien auf der Körperoberfläche gekennzeichnet, die eine Muskelnprojektion darstellen, die an der Bewegung teilnimmt und ein System der Wellenleiter bildet, in deren Wegen sich Muskelkräfte verbreiten, die die biomechanische Lymph- und Blutbewegung fördern. Die Durchführung der Wellenmassage bei dem vorgeschlagenen Verfahren gemäß den biomechanischen Meridianen lässt Massagehandgriffe zu, die entweder ungeradlinig oder gleichzeitig nach mehreren Richtungen einer komplizierten Konfiguration und bedeutenden Ausdehnung, die mit der Körperlänge vergleichbar ist, oder in entgegengesetzten Richtungen ausgeführt werden. Zum Beispiel wechselt die Welle mit einer Geschwindigkeit von 0,01 m/s, die den Lymphkreislauf stimuliert und die sich von dem Distalteil des Körpers zum Proximalteil bewegt, mit der Welle der entgegengesetzen Richtung für die Stimulierung des Arterienumlaufs mit einer Geschwindigkeit von 8 m/s ab, was bei jeder Art der Apparatmassage, darunter auch die manuelle Massage, unerreichbar ist.

Die Einwirkungen mit einer tiefen Temperatur von 0°C lässt bei dem Vibrationsprozess mit einem großen Verhältnis eine hohe Geschwindigkeit der Abkühlung des Körperteils ohne Erniedrigung der gesamten Temperatur des Menschenkörpers zu. Die Einwirkung mit einer hohen Temperatur von 90°C lässt während des Vibrationsprozess mit einer biomechanischen Resonanzfrequenz und einem großen Verhältnis den Körperteil ohne Brandwundentrauma und totale Temperaturerhöhung des Menschenkörpers erwärmen.

Die Wirkung der einzelenen Vibratoden auf den Menschenkörper hängt einmal vom eigenen mechanischen Impuls ab und wird zum anderen durch die Dynamik der Nachbarvibratoden und die Kraft zwischen diesen sowie gleichfalls durch die Steuerkraft für die Verschiebungen der Vibratoden bestimmt. Durch die Verbindungssteuerung zwischen den Vibratoden lässt sich die Druckkraft der Massage einstellen, die für die Durchführung zum Beispiel von Streichhandgriffen notwendig ist. Die Messung der Masseurkraft während der Massage und auch Untersuchungen für die Bestimmung der optimalen Kraft der Einwirkung des Vibratodes auf den Menschenkörper bei dem vorgeschlagenen Verfahren haben den Bereich des spezifischen Drucks festgelegt. Der maximale spezifische Druck ist $4 \times 10^5$ Pa. Er übersteigt um $3,0 \times 10^5$ Pa den atmosphärischen Luftdruck und wird nach der Methode der tonischen Massage (Sportmassage) bestimmt, bei der sich die Massagehandgriffe mit größerer Kraft als zum Beispiel bei der Kosmetik oder Kurmassage ausgeführt werden. Der minimale spezifische Druck wird aus den Bedingungen der Wirksamkeit der Unterdruckwelle bestimmt, die durch den Stand des Blutzuflusses im massierten Körperteil gemessen wird. Als Ergebnis der Untersuchungen wurde festgestellt, dass ein Trauma der Muskeln bei der Massage nicht auftritt, wenn der minimale Druck $0,5 \times 10^5$ Pa und die Frequenz der mechanischen Impulse mehr als 1 Hz beträgt.

Es ist bekannt, dass die Druckkraft von dem Produkt der Normalen zur Oberflächenkraft und des Reibungskoeffizienten abhängt, der in diesem Fall von der Auswahl des an den Körper gedrückten, elastischen Mediums abhängt. Bei der vorliegenden Erfindung ergibt sich ein Reibungskoeffizient von 0,05 bis 0,45, durchschnittlich 0,25. Die minimale Druckkraft wird aus den Massagebedingungen der Gesichtsmuskeln festgestellt, bei denen die Normalkraft 0,4 h beträgt:

$$f_{\text{min. Verschiebung}} = 0,25 \times 0,4 = 0,1H$$

Bei der Wellenmassage des Körpers kann der normale maximale Druck 400 H sein. Die maximale Druckkraft beträgt in diesem Fall aus:

$$f_{\text{max. Verschiebung}} = 0,25 \times 400 = 100H$$

Die einzelne, modulierte Welle kann man als Gruppeneinheit, als Wellenzug oder als eine Reihenfolge der mechanischen Impulse betrachten, die sich durch den Menschenkörper mit einer Gruppengeschwindigkeit fortpflanzen. Dabei kommt es während der Fortpflanzung im Wellenzug (im Paket) zur Amplituden- und Frequenzveränderung der mechanischen Impulse, das heißt, es wird eine Amplituden- und Frequenzmodulation der Schwingungen beobachtet. Es ist bekannt, dass bei jedem traditionellen Modulationsverfahren der Schwingungen die Geschwindigkeit der Amplituden- und Frequenzänderungen hinreichend klein sein soll, damit der Modulationsparameter sich während der Schwingungsperiode nur unwesentlich verändert. Bei dem vorgeschlagenen Verfahren wird diese Toleranz nicht ausgenutzt, wodurch sich die Massage unter der Wirkung der Organismusrezeption zur Umgebung durchführen lässt. Die Parameter der modulierten Schwingungen wurden auf folgende Weise bestimmt. Die Frequenz der modulierenden Schwingungen beträgt:

$$f = 1/(N \times T_z),$$

wobei das Verhältnis des modulierenden Signals (das Verhältnis der Zeit des Wellendurchgangs durch alle Vibratoden zur Zeit der Schwingung einer Vibratode durch folgenden Ausdruck gekennzeichnet ist:

$$q = (N \times T_z)/T_z = N,$$

wobei $N$ = die Zahl der Vibratode und $T_z$ = die Zeit der Schwingungen einer Vibratode ist.

Die Anzahl der Vibratoden zur Wellenbildung auf der ganzen Länge des biomechanischen Glieds soll nicht weniger als 4 und in der Regel nicht mehr als 32 sein. die Vibrationsdauer, bei der eine Organismusadaption zur mechanischen Schwingungen nicht beginnt, beträgt 8 Sekunden. Dann ist:

$$f_{min} = 1/(N_{max} x T_{Zmax}) = 1/(32x8) = 0,004 Hz$$

$$f_{max} = 1/(N_{min} x T_{Zmin}) = 1/(4x0,25) = 1 Hz$$

Dabei ändert sich das Verhältnis von 4 bis 32, und insgesamt ist der Anzahl der Vibratoden gleich, die an der Wellenbildung teilnehmen. In Bezug auf die schwingungstragenden Einzelwellen wurde festgestellt, dass durch sie die Blutversorgung bei der Massage verbessert werden kann, wenn sie als eine Reihenfolge von mechanischen Impulsen mit der Frequenz von 1 bis 40 Hz und dem Verhältnis von 1,6 bis 6 dargestellt werden. Bei dem vorgeschlagenen Verfahren hat das modulierende Signal eine Impulsform, die Resultierende aber wird wie ein Schwingungszug dargestellt; in Bezug auf diesen wird eine Frequenzmodulation durch das Signal in jeder Form verwirklicht, darunter auch durch ein Sinussignal. Dabei verändert sich die Schwingungsfrequenz nach dem Gesetz:

$$f = f_0 + f_D x sin(2xqxvxt),$$

f = Schwingungsfrequenzen, Hz
$f_0$ = Schwingungsfrequenzen am Ausgangspunkt der Zeit, Hz
$f_D$ = Frequenzabweichung, Hz
v = Modulationsfrequenz, Hz
t = Zeit

Mit Rücksicht darauf, dass die Begrenzung der Geschwindigkeitsveränderung der Frequenz bei dem vorgeschlagenen Verfahren vermindert ist, kann die Modulationsfrequenz den Wert im ganzen Bereich der möglichen Frequenzen annehmen, das heißt von 0,001 bis 40 Hz, und die Modulationsfrequenz entspricht dem Frequenzbereich der modulierenden Schwingung, das heißt 0,004-1 Hz. Der untere Wert der Frequenzabweichung ist gleich 0,001 und wird aus dem Mangel an physiologischen Wirkungen bestimmt, die mit der Frequenzabweichung unter 0,001 verbunden sind. Diese Einstellung schließt eine schnelle Adaptation der physiologischen Organismusfaktoren an das Verfahren der Biomechanotherapie aus, und es lassen sich eine Massage sowohl mit einer Welle von der Dauer 1 Minute als auch eine Gemeinmassage von der Dauer bis 10 Stunden durchführen. Mit einer Massage von einer Dauer von weniger als 1 Minute lassen sich keine physiologischen Verschiebungen im Organismus hervorrufen. Eine dauernde Biomechanotherapie hat erstrangige Bedeutung bei der Wiederherstellung der physischen Arbeitsfähigkeit der Fachsportler und bei der Vorbeugung der Nachoperationskomplikationen, sowie bei der Kosmonautenarbeit und den Bedingungen der Schwerelosigkeit. Die durchgeführten Untersuchungen haben gezeigt, dass sich durch die Biomechanotherapie mit einer Dauer von 10 Stunden die physischen Fähigkeiten von Sportlern nach großen physischen Belastungen bedeutend wiederherstellen und bei Kranken eine Nachnarkosedepression und auch begleitende Bronchien-, Lungenkomplikationen in der Nachoperationsperiode vermeiden lassen.

Das vorgeschlagene Verfahren zur Biomechanotherapie wird anhand von Zeichnungen näher erläutert. Es zeigen

Fig. 1    ein elektronisch-pneumatisches Schema eines Geräts zur Verwirklichung des vorgeschlagenen Verfahrens mit Hilfe von 8 Vibratoden,

Fig. 2    ein Schema der Lage der Vibratoden bei der Kurmassage der Rückenmuskeln,

Fig. 3    ein Schema der Regelung der elastischen Verbindung zwi- schen den Vibratoden und

Fig. 4    Wellentypen, die sich für das vorgeschlagene Verfahren eignen.

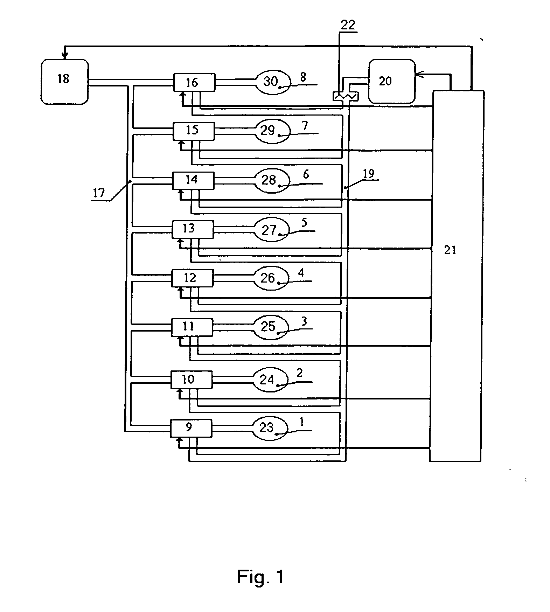

Das elektronisch-pneumatische System zur Verwirklichung der vorgeschlagenen Biomechanotherapie (Fig. 1) enthält Vibratoden 1-8, deren Arbeitsräume durch Luftleitungen mit entsprechenden dreilinearen, pneumatischen Ventilen 9-16 verbunden sind. Diese Ventile werden durch eine Luftleitung 17 mit einer geregelten Vakuumquelle 18 und durch eine Luftleitung 19 mit einer geregelten Druckluftquelle 20 verbunden. Die elektromagnetischen, pneumatischen Ventile und eine Druck- und Vakuumunterstützung werden mit Hilfe eines Rechners 21 gesteuert. Zur Erwärmung und Abkühlung der den Vibratoden zugeführten Luft dient eine Klimaanlage 22. Die programmierbare Ventilsteuerung wird durch die Zuführung eines elektrischen Signals zur Ventilwicklung verwirklicht. Der Arbeitsraum jeder Vibratode wird dabei mit der Luftdruckquelle verbunden und bei Stromausfall in den Ventilumwicklungen verbindet die Vibratode sich entweder mit der Vakuumsquelle oder mit der Atmosphäre. Alle Vibratoden besitzen auch Laserstrahlungsquellen 23-30.

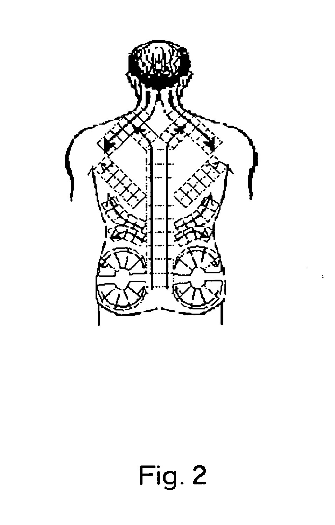

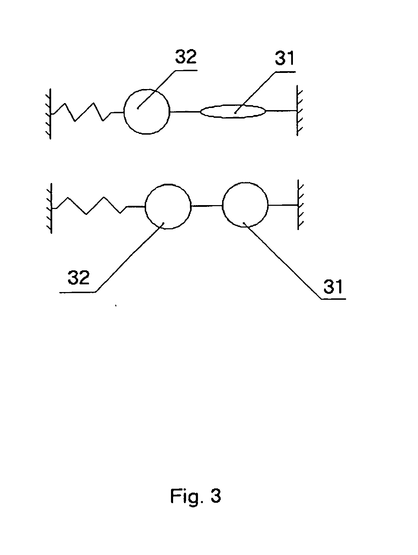

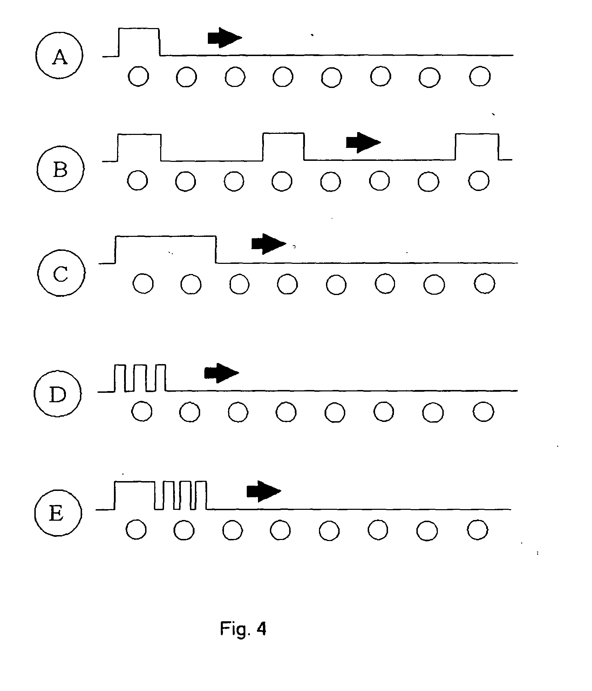

Mit Hilfe der in Fig. 1 gezeigten Anlage wird das Biomechanotherapieverfahren auf folgende Weise verwirklicht. Als Beispiel soll die Biomechanotherapie der Rückenmuskeln betrachtet werden, die mit Hilfe der Vibratoden realisiert werden kann, die auf dem Menschenkörper entsprechend der Fig. 2 angeordnet sind. Die Drehachse jeder Vibratode steht zum biomechanischen Meridian senkrecht. Wenn sich die Welle bei dem vorgeschlagenen Biomechanotherpieverfahren unterbrochen bewegt, so entspricht oft der biomechanische Meridian den Richtungen der Grundmassagebewegungen bei der manuellen Massage. Für die Regelung der elastischen Verbindung sind einige Hülle mit sehr kleinen Ausmaßen als Massagehüllen hergestellt worden, und beim Anfüllen wirken sie nicht nur auf den Menschenkörper, sondern haben auch die Funktion eines Gummiantriebs, indem sie Nachbarvibratoden fördern. Der Regelungsaufbau der elastischen Verbindung ist in Fig. 3 dargestellt. Während des Anfüllens der Hülle 31 (Fig. 3) bewegt sich die Massagevibratode 32, das heißt, es treten Druckkräfte auf. Mit Hilfe der beschriebenen Anlage können mehrere Wellentypen realisiert werden (Fig. 4):

A-Wellen, die durch Einwirkung von einzelnen Impulsen der 1-8 Vibratoden gebildet sind,

B-Wellen, die durch Einwirkung von Einzelimpulsen gebildet sind, indem sie durch die Vibratoden 1-3 abgeleitet sind.

C-Wellen, die aus zwei verdoppelten mechanischen Impulsen bestehen,

D-Wellen, die durch Einwirkung der einzelnen Vibratoden wie eine Reihenfolge der mechanischen Impulse gebildet sind, und

E-Wellen, die aus zwei verdoppelten Impulsen bestehen, wobei der zweite aus hochfrequenzten Trägerschwingungen besteht.

Je nach der Lage der Vibratode wird die Frequenz der mechanischen Impulse gewählt. Natürlich wird die Frequenz während der Wellenbewegung von Vibratode zu Vibratode verändert. Gibt es eine Welle, die aus einzelnen Impulsen entstanden ist, drückt sich die Frequenzmodulation in der Wellengeschwindigkeitsveränderung je nach der Bewegung von Vibratode zu Vibratode aus.

Als Beispiel kann das Verfahren zur Biomechanotherapie der Rückenmuskeln (des Brustteils der Wirbelsäule) genommen werden. Als Grundhandgriffe der Massage werden folgende verwendet: Streichen und Aufwärmung. Als Ergebnis der Untersuchungen ist festgestellt worden, dass dem Streichen die Wellen A05, B05, C05 und E05 mit einer Geschwindigkeit von 0,5 m/s entsprechen. Der Aufwärmung entspricht die Welle C0166, die durch verdoppelte Impulse mit einer Geschwindigkeit von 0,166 m/s gebildet ist. Der Aufwärmung entspricht auch die Welle D0125, die sich mit einer Geschwindigkeit von 0,125 m/s einer Frequenz von 5 Hz, einem Verhältnis von 2, einer Vibrationszeit jeder Vibratode von 4 s, einer Modulationsfrequenz von 0,5 Hz und einer Frequenzabweichung von 20 Hz fortpflanzt. Die Massage wird nach dem Schema A05, A05, C05, E05, A05, C0166, A05, D0125, A05 C05 und A05 durchgeführt, was die Reihenfolge der Wellenwirkung betrifft. Es wird betont, dass während der Biomechanotherapie nach jedem Handgriff der Kurmassage in der Regel der Streichhandgriff mit Hilfe der Welle A05 folgt. Dabei wird eine Wärmeregelung der Vibtatode durch die Luftzufuhr mit unterschiedlichen Temperaturen verwirklicht; die Wärmebehandlung dient dem thermobiomechanischen Tonus der Muskeln. Die Vibratoden können entweder Laserstrahlungsquellen besitzen oder aus durchsichtigen, elastischen Hüllen gefertigt sein, außerhalb derer sich ein Strahler befindet, der durch einen faseroptischen Lichtleiter mit eigener Laserstrahlungsquelle verbunden ist, deren Beleuchtungsstärke phasenrichtig mit mechanischen Schwingungen der Impulse synchronisiert ist, die Quellen dienen insgesamt zur Bildung einer einzelnen Lichtwelle. Die Wellenbewegung der einzelnen Vibratode kann nicht nur einen Druck ausüben, sonder auch Unterdruckwellen hervorrufen. Das Verdünnen in der einzelnen Vibratode und das Vorhandensein des Drucks in Nachbarvibratoden bilden auf dem Körper ein Verdünnungsbereich. Bei der Bewegung des Verdünnungsbereichs auf dem Menschenkörper wird eine Unterdruckwelle gebildet.

Es ist bekannt, dass die Geschwindigkeit eines Blutumlaufs von der Körpertemperatur abhängt. Es muss darauf hingewiesen werden, dass die Temperaturerhöhung einer physischen Arbeit zur Erhöhung der Hemodynamik beiträgt. Für die Wirksamkeitserhöhung der Biomechanotherapie wird die der Vibratode zugeführte Luft erwärmt. Die größte Wärmeübertragung von der Massagevibratode zum Menschenkörper erfolgt im Moment des Anfüllens. Auf diese Weise werden auf dem Menschenkörper Temperaturwellen gebildet, und infolge dessen entstehen ein Temperaturabfall in den massierten Muskeln und als Folge ein thermobiomechanisches Tonisieren der Muskeln. Als Ergebnis des letzten ergibt sich eine Verbesserung der Muskelkontraktionsfähigkeit durch die Thermostimulierung des Organismus synchron mit Vibration der Muskeln, die der biomechanischen Resonanzwirkung unterliegen. Das Vorhandensein des Ausstrahlers in der Vibratode lässt eine Blutbestrahlung durch die Haut zu. Die Strahler wirken durch die elektromagnetische Strahlung auf Reflexzonen und biologisch aktive Punkte während der Wellenbiomechanotherapie invasionsfrei. Die Phasensynchronisierung der mechanischen Schwingungen der Vibratoden und die Strahlungsintensität lassen eine Erhöhung der Wirksamkeit der biologischen Stimulierung der Organismuslebenstätigkeit insgesamt zu. Das ist durch die Synchronisierung der Makro (Massage)- und Mikro (Ausstrahlungs)-Einwirkungen bedingt.

Beispiel 1

Ein Patient T., 42 Jahre alt, hat sich im klinischen Gebietskrankenhaus vom 28.07.2000 bis 20.09.2000 ärztlich behandeln lassen. Nach einer totalen, komplexen Untersuchung wurde eine klinische Diagnose gestellt: verbreitete Osteochondrose des Kreuzteils der Wirbelsäule mit einem ausgeprägtem Wurzelsyndrom. Für die Durchführung der Biomechanotherapie wurde der Patient waagerecht gelegt. Unter dem Rücken des Patienten wurde eine Massagevorrichtung gelegt (ein Teppich) mit Vibratoden verschiedener Durchmesser. Für eine Kur wurden 10 Behandlungen verschrieben. Bei den ersten 5 Behandlungen wurde die Kurwirkung ausgenutzt, bei den letzten 5 wurde die intensivere Sportwirkung ausgenutzt. Der Zustand des Kranken nach der Durchführung der Wellenbiomechanoeinwirkung wurde viel besser. Das Schmerzsyndrom im Bereich der Wirbelverletzungen wurde praktisch völlig beseitigt, und die Bewegungsfunktion der Wirbelsäule wurde wesentlich verbessert. Komplikationen, Schmerzen und unangenehme Empfindungen gab es bei der Durchführung der Kurmassage nicht. Damit ist bewiesen, dass die Wellenbiomechanotheprapie ein hinreichend wirksames Einwirkungsverfahren bei der Komplexkur der Kranken mit verbreiteter Osteochondrose ist. Eine besondere Bedeutung hat diese Methode bei der Kur der Kranken mit polyvalenter Allergie gegen Medikamente oder beim Vorhandensein von Gegenanzeigen zu den physisch-therapeutischen Einwirkungen, insbesondere bei der manuellen Therapie.

Beispiel 2

Die Kranke M., 59 Jahre alt, hat sich in der flebologischen Klinikabteilung der Hospitalchirurgie vom 18.09.2000 bis 02.10.2000 ärztlich behandeln lassen. Nach der totalen komplexen Untersuchung wurde eine klinische Diagnose gestellt: Der Krankheitszustand entstand nach einer radikalen Mastektomie rechts im Jahre 1991: die wiederholte Lymphostase des rechten oberen Gliedes III Grad. Bei der Untersuchung wurde eine bedeutende Volumenvergrößerung des rechten oberen Gliedes auf dem proximalen Gelenk festgestellt; der Durchmesser des Kreises betrug 35,5 sm, des oberen Drittels des Unterarms 37 sm und des oberen Drittels der Schulter 37 sm. Bei der Durchführung der Biomechanotherapie wurde eine spezielle Manschette für das obere Glied, die Vibratoden besaß, von der Mitte des Handrückens in Richtung der Massagewellenbewegung der Finger angelegt. Die Manschette wurde möglichst dicht an den Leib angelegt. Es ist empfehlenswert, eine elastische Binde auf das Glied für die Durchführung der Biomechanotherapie anzulegen. Das obere Glied wurde nach oben gelegt, da diese Lage physiologischer für die Durchführung dieser Prozedur ist. Für eine Kur wurden 10 Behandlungen vorgeschrieben. Die ersten 7 Behandlungen wurden unter Kuraufsicht durchgeführt, weitere 3 unter Sportaufsicht. Der Zustand der Kranken ist nach der Durchführung der Wellenbiomechanotherapie viel besser geworden: das Gliedanschwellen nahm ab und der Zustand der Hautfläche verbesserte sich. Die Kreislänge auf dem proximalen Gelenk betrug 29,5 sm, das obere Drittel des Unterarm 33,5 sm und das mittlere Drittel der Schulter 33 sm. Komplikationen, Schmerzen oder unangenehme Empfindungen bei der Durchführung der Behandlung gab es nicht. Abschließend kann gesagt werden: die Wellenbiomechanotherapie ist eine hinreichend wirksame Einwirkungsmethode bei der komplexen Heilung der mit wiederholten Lymphostasen behafteten Gliedmaßen auch in der Voroperationsvorbereitung und in der Nachoperationsrehabilitation dieser Kategorie von Kranken.

Mit dem vorgeschlagenen Verfahren zur Biomechanotherapie lässt sich die Wirksamkeit der Kur- und Sportmassage erhöhen und lassen sich die Ergebnisse der Komplexheilung der unterschiedlichen Erkrankungen verbessern. Die Wellenbiomechanotherapie wirkt im Frequenzbereich der biomechanischen Muskelresonanz von 5 bis 20 Hz, indem sie optimale Bedingungen für eine vollwertige Wiederherstellung des Nerven- und Muskelsystems bietet. Indem die Wellenbiomechanotherapie nach dem Prinzip der einzelnen Wanderwelle arbeitet, erhöht sie den priperischen Blutkreislauf indem sie die Arbeit des Herz-Gefäßsystems erleichtert und indem sie die reologische Eigenschaften des Blutes und Blutkreislaufs aller Organe und deren Funktionszustand verbessert. Dank der aktiven Stimulierung des Stoffwechsels beschleunigt sich die Wellenbiomechanotherapie die Wiederherstellung in Zellen und Geweben und verstärkt die Entfernung von Zerfallprodukten aus dem Organismus auf natürlichem Wege. Durch die Verwendung der Wellenbimechanotherapie lässt sich das Allgemeinbefinden und der Lebenstonus verbessern, die Arbeitsfähigkeit steigern, die Elastizität der Muskeln und die Beweglichkeit von Sehnenbändern erhöhen, eine Stagnationserscheinung und eine Wassergeschwulst ausschließen. Es ist wichtig, dass die oben genannten Ergebnisse durch eigene Organismusresourcen erreicht werden, ohne künstliche Stimulantien, Medikamente und erschöpfendes Trainieren. die vorliegende Erfindung kann eine breite Verwendung in Medizin, Sport und Kosmonautik finden.

**EP 1 364 637 B1**

**Patentansprüche**

1. Vorrichtung zur biomechanischen Wellentherapie mit pneumatischen Vibratoden (1 bis 8), welche Arbeitsräume aufweisen, die über Luftleitungen mit elektromagnetisch steuerbaren pneumatischen Dreiwegeventilen (9 bis 16) verbunden sind, welche Dreiwegeventile (9 bis 16) über eine erste Luftleitung (17) mit einer geregelten Unterdruck- bzw. Vakuumquelle (18) und über eine zweite Luftleitung (19) mit einer geregelten Druckluftquelle (20) verbunden sind, wobei die elektromagnetisch steuerbaren pneumatischen Dreiwegeventile (9 bis 16), die geregelte Unterdruck- bzw. Vakuumquelle (18) und die geregelte Druckluftquelle (20) mittels eines Rechners (21) programmierbar gesteuert sind, sowie ferner mit mindestens einer Laserstrahlungsquelle (23 bis 30) je Vibratode (1 bis 8) und einer Einrichtung (22) zur Temperierung der den Vibratoden (1 bis 8) zugeführten Luft.

2. Vorrichtung nach Anspruch 1,
   **dadurch gekennzeichnet,**
   **dass** die Laserstrahlungsquellen (23 bis 30) Bestandteile der pneumatischen Vibratoden (1 bis 8) sind.

3. Vorrichtung nach Anspruch 1,
   **dadurch gekennzeichnet,**
   **dass** die Vibratoden (1 bis 8) aus die Arbeitsräume umschließenden, durchsichtigen elastischen Hüllen gefertigt sind, außerhalb derer sich ein Strahler befindet, der durch einen faseroptischen Lichtleiter mit einer eigenen Laserstrahlungsquelle (23 bis 30) verbunden ist.

4. Vorrichtung nach Anspruch 1, 2 oder 3,
   **dadurch gekennzeichnet,**
   **dass** die Beleuchtungsstärke der Laserstrahlungsquellen (23 bis 30) phasenrichtig mit den mechanischen Schwingungen der Vibratoden (1 bis 8) synchronisiert ist.

5. Vorrichtung nach einem der Ansprüche 1 bis 4,
   **gekennzeichnet durch**
   eine Wärmeregelung der Vibratoden (1 bis 8) **durch** eine Luftzufuhr mit unterschiedlichen Temperaturen.


**Claims**

1. A device for wave biochemical therapy, having pneumatic vibratodes (1 through 8), which have work chambers that communicate via air lines with electromagnetically controllable pneumatic three-way valves (9 through 16), which three-way valves (9 through 16) communicate with a regulated underpressure or vacuum source (18) via a first air line (17) and with a regulated compressed air source (20) via a second air line (19), wherein the electromagnetically controllable pneumatic three-way valves (9 through 16), the regulated underpressure or vacuum source (18), and the regulated compressed air source (20) are controlled programmably by means of a computer (21), and further having at least one laser radiation source (23 through 30) per vibratode (1 through 8) and one device (22) for tempering the air supplied to the vibratodes (1 through 8).

2. The device as defined by claim 1,
   **characterized in that**
   the laser radiation sources (23 through 30) are components of the pneumatic vibratodes (1 through 8).

3. The device as defined by claim 1,
   **characterized in that**
   the vibratodes (1 through 8) are made from transparent elastic sheaths surrounding the work chambers, outside which there is an emitter which is connected to its own laser radiation source (23 through 30) by means of a fiber optical waveguide.

4. The device as defined by claim 1, 2 or 3,
   **characterized in that**
   the luminosity of the laser radiation sources (23 through 30) is synchronized in-phase with the mechanical vibrations of the vibratodes (1 through 8).

**5.** The device as defined by one of claims 1 through 4,
**characterized by**
a heat regulation of the vibratodes (1 through 8) by means of a supply of air at different temperatures.

## Revendications

**1.** Dispositif de thérapie biomécanique par ondes, avec des vibratodes pneumatiques (1 à 8) qui présentent des espaces de travail qui sont reliés par des conduites d'air à des vannes trois voies (9 à 16) pneumatiques à commande électromagnétique, lesquelles vannes trois voies (9 à 16) sont reliées par une première conduite d'air (17) à une source de dépression ou de vide régulée (18) et par une deuxième conduite d'air (19) à une source d'air comprimé régulée (20), les vannes trois voies (9 à 16) pneumatiques à commande électromagnétique, la source de dépression ou de vide régulée (18) et la source d'air comprimé régulée (20) étant commandées de manière programmable au moyen d'un ordinateur (21), ainsi qu'avec au moins une source de rayonnement laser (23 à 30) par vibratode (1 à 8) et un dispositif (22) pour réguler la température de l'air amené aux vibratodes (1 à 8).

**2.** Dispositif selon la revendication 1,
**caractérisé en ce**
**que** les sources de rayonnement laser (23 à 30) sont des composants des vibratodes pneumatiques (1 à 8).

**3.** Dispositif selon la revendication 1,
**caractérisé en ce**
**que** les vibratodes (1 à 8) sont réalisées à partir d'enveloppes élastiques transparentes entourant les espaces de travail, à l'extérieur desquelles se trouve un émetteur de rayonnement qui est relié par un guide de lumière à fibre optique à une source de rayonnement laser (23 à 30) dédiée.

**4.** Dispositif selon la revendication 1, 2 ou 3,
**caractérisé en ce**
**que** l'intensité lumineuse des sources de rayonnement laser (23 à 30) est synchronisée de manière à être en phase avec les vibrations mécaniques des vibratodes (1 à 8).

**5.** Dispositif selon une des revendications 1 à 4,
**caractérisé par**
une régulation thermique des vibratodes (1 à 8) par une amenée d'air à différentes températures.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4231355 A
- US 1795889 A
- WO 0067693 A
- DE 3905517 C1
- US 604211 A
- RU 2045972
- RU 2161517